**Europäisches Patentamt**

**European Patent Office**

**Office européen des brevets**

(19)

(11) Numéro de publication : **0 400 736 B1**

# (12) FASCICULE DE BREVET EUROPEEN

(45) Date de publication du fascicule du brevet :
**11.08.93 Bulletin 93/32**

(51) Int. Cl.$^5$ : **C07C 21/06,** C07C 17/38

(21) Numéro de dépôt : **90201322.6**

(22) Date de dépôt : **25.05.90**

(54) **Procédé d'épuration de chlorure de vinyle.**

(30) Priorité : **02.06.89 FR 8907503**

(43) Date de publication de la demande :
**05.12.90 Bulletin 90/49**

(45) Mention de la délivrance du brevet :
**11.08.93 Bulletin 93/32**

(84) Etats contractants désignés :
**AT BE CH DE DK ES FR GB GR IT LI LU NL SE**

(56) Documents cités :
**US-A- 2 356 562**

(73) Titulaire : **SOLVAY (Société Anonyme)
Rue du Prince Albert, 33
B-1050 Bruxelles (BE)**

(72) Inventeur : **Strebelle, Michel
Rue Sombre 84
B-1150 Bruxelles (BE)**

(74) Mandataire : **Nichels, William et al
Solvay Département de la Propriété
Industrielle Rue de Ransbeek, 310
B-1120 Bruxelles (BE)**

EP 0 400 736 B1

## Description

La présente invention concerne un procédé d'épuration de chlorure de vinyle en composés du type ester et plus particulièrement en composés esters, tels que l'acétate de vinyle, possédant une insaturation dans la molécule.

Il est connu que le chlorure de vinyle utilisé dans l'industrie est un composé qui peut être obtenu selon diverses méthodes de fabrication qui ont été optimalisées de façon à engendrer un composé dont la pureté est élevée et, en tous cas, suffisante pour les utilisations auxquelles ce composé est habituellement destiné et tout particulièrement à son homopolymérisation.

Un tout autre problème, apparu récemment dans l'industrie, pour des raisons économiques et d'environnement, est le problème posé par l'épuration de chlorure de vinyle résiduaire, non transformé ou issu de procédés industriels dans lesquels il a été mis en oeuvre. Dans ce contexte, le problème posé par la séparation du chlorure de vinyle, d'esters tels que l'acétate de vinyle, occupe une place particulière puisqu'il concerne la séparation de deux comonomères résultant d'une opération de copolymérisation dans laquelle, faute de solution satisfaisante au niveau du recyclage des deux comonomères, on acceptait de perdre tout ou partie des deux monomères par des opérations de destruction non sélectives telles que le brûlage.

On a maintenant trouvé un nouveau procédé qui ne possède pas les inconvénients précités et qui permet, moyennant la destruction du composé ester uniquement, de récupérer du chlorure de vinyle très pur, ce qui permet d'en faire une utilisation identique à celle d'un chlorure de vinyle directement issu d'une unité de fabrication.

La présente invention concerne à cet effet un procédé d'épuration de chlorure de vinyle en composés de type ester présentant une structure d'esters d'énol, caractérisé en ce que le chlorure de vinyle subit un traitement comprenant une étape de lavage alcalin suivie ou concomitante à une étape de traitement avec un hydrure et de préférence avec un borohydrure.

Les composés de type ester présents généralement dans le chlorure de vinyle sont des composés esters possédant dans leur molécule une insaturation qui les rend aptes à copolymériser par voie radicalaire avec le chlorure de vinyle. Un composé de ce type ayant conduit à de bons résultats est l'acétate de vinyle. Il est toutefois évident que le procédé de l'invention s'applique aisément à tout type d'ester insaturé présentant une structure d'ester d'énol qui peut se retrouver pour une quelconque raison dans le chlorure de vinyle.

L'étape de lavage alcalin du chlorure de vinyle impur peut être réalisée en principe avec tout agent basique connu, mais est habituellement réalisée avec une solution aqueuse d'une base dérivée de métaux alcalins ou alcalino-terreux. De préférence, ce traitement est effectué avec un hydroxyde de ces métaux. Enfin, de bons résultats ont été obtenus avec de l'hydroxyde de sodium. Quoique la présente invention n'entend en aucune façon être limitée ou être tributaire d'une quelconque explication ou théorie scientifique, il est fort probable que le traitement alcalin transforme l'entièreté du composé ester en un sel et un aldéhyde. Ainsi, plus particulièrement dans le cas de l'acétate de vinyle il se formerait à ce stade de l'acétate de sodium et de l'acétaldéhyde lorsque le procédé est exécuté à l'intervention d'hydroxyde de sodium.

Lorsque l'étape de lavage alcalin est effectuée avec une solution aqueuse d'hydroxyde de sodium, la concentration en NaOH est généralement comprise entre 0,01 M/l et 10 M/l et de préférence entre 0,1 M/l et 2 M/l.

L'étape de lavage alcalin est suivie d'une étape de traitement du chlorure de vinyle avec un hydrure et plus particulièrement un borohydrure tel que le borohydrure de sodium, ce qui a comme conséquence d'épurer le chlorure de vinyle probablement par élimination d'aldéhydes tels que l'acétaldéhyde résiduaire présent dans le milieu.

Le traitement au borohydrure de sodium présente en outre l'avantage de transformer des aldéhydes tels que l'acétaldéhyde en alcools, par une réaction de réduction pouvant être effectuée en milieu alcalin. Dès lors, il devient techniquement possible de condenser le lavage alcalin de la première étape et le traitement au borohydrure en une seule opération. Une telle opération ne constitue actuellement toutefois pas la solution préférée industriellement, mais ceci est uniquement dû au prix élevé des borohydrures. En attendant, une solution ayant donné de bons résultats consiste à opérer en deux étapes distinctes, tout en renvoyant une partie ou la totalité de l'effluent aqueux de la deuxième étape, qui contient immanquablement une partie de borohydrure, au réacteur de la première étape dans lequel a lieu le lavage alcalin. Cette solution présenterait quant à elle l'avantage d'une réduction des fonctions carbonyles des produits lourds, issus d'une éventuelle aldolisation des aldéhydes formés lors du lavage alcalin de la première étape, et de réduire les salissages liés obligatoirement à la présence de tels produits lourds.

Lorsque le procédé de l'invention fait intervenir le borohydrure de sodium, celui-ci est généralement mis en oeuvre en solution aqueuse basique telle qu'une solution d'hydroxyde de sodium dont la concentration peut varier entre 0,1 M/l et 10 M/l et de préférence entre 1 et 5 M/l. la concentration en NaBH$_4$ dans cette solution

2

alcaline est en général comprise entre 0,01 M/l et la limite de solubilité du $NaBH_4$ dans cette solution alcaline; de préférence la concentration en $NaBH_4$ est toutefois comprise entre 0,1 et 1 M/l.

Outre les étapes précitées, dont la séquence opérationnelle est essentielle pour l'obtention de chlorure de vinyle exempt d'esters, le procédé de l'invention peut prévoir d'autres étapes selon les conditions particulières qui ont conduit à un tel chlorure de vinyle.

Le procédé de l'invention, lorsqu'il est réalisé à l'intervention de borohydrure de sodium sur du chlorure de vinyle issu d'une fabrication de copolymérisation de chlorure de vinyle et d'acétate de vinyle, peut être effectué dans des conditions de température comprises entre 0° et 70°C et de préférence entre 0° et 10°C pour ce qui concerne l'étape de lavage alcalin et entre 10° et 70°C et de préférence entre 15 et 35°C pour ce qui concerne l'étape de réduction.

En ce qui concerne les conditions de pression, celles-ci ne sont pas critiques et sont généralement comprises entre 1 et 10 bars pour les deux étapes. Habituellement, les deux étapes sont réalisées à la pression atmosphérique.

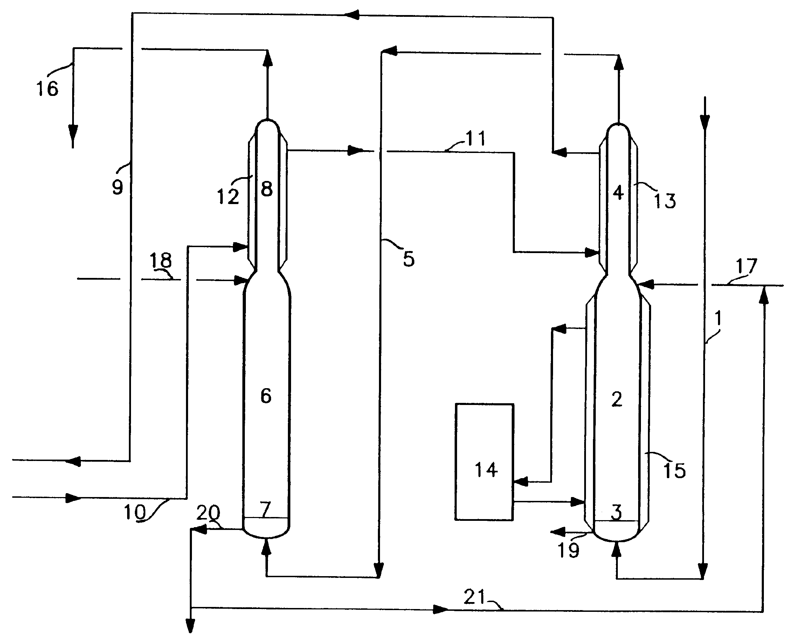

Le procédé de l'invention peut être réalisé dans toute installation permettant d'effectuer les étapes décrites ci-dessus et comportant des colonnes noyées, arrosées, munies éventuellement d'un garnissage et capables de fonctionner soit en batch soit en continu. Un schéma d'une installation ayant donné de bons résultats est repris à la figure 1 et comprend :

- une conduite 1 par laquelle est introduit dans le système du chlorure de vinyle gazeux contenant l'ester à éliminer,
- un premier réacteur 2, équipé d'une double enveloppe 15, dans lequel est introduit le chlorure de vinyle impur via la conduite 1. Ce réacteur 2 est avantageusement équipé d'un dispositif de répartition des gaz dans le liquide 3 et d'un condenseur 4 permettant respectivement d'effectuer un bon barbotage des gaz et de retenir les produits liquides à la température de lavage alcalin,
- une conduite 5 reliant le condenseur 4 du réacteur 2 à un deuxième réacteur 6 destiné à effectuer le traitement au borohydrure,
- ce réacteur 6 est de nouveau avantageusement équipé d'un dispositif de répartition des gaz dans le liquide 7 et d'un condenseur 8 dont les fonctions sont identiques à celles du dispositif 3 et du condenseur 4,
- le chlorure de vinyle pur est ensuite envoyé par la conduite 16 au séchage suivi d'une recompression et d'une liquéfaction afin de permettre le stockage du chlorure de vinyle pur,
- enfin l'installation est complétée par un circuit de réfrigération permettant respectivement, via les conduites 10 et 11, de refroidir par les doubles enveloppes 12 et 13 les condenseurs 4 et 8 et, par un circuit de réfrigération indépendant 14, d'assurer le refroidissement de la double enveloppe 15 du réacteur de lavage alcalin 2.

Les réacteurs 2 et 6 peuvent en outre être équipés pour fonctionner en continu ou en discontinu. A cet effet, des conduites 17 et 18 sont prévues pour les alimenter respectivement en solutions aqueuses de NaOH et de NaOH et de borohydrure. Par ailleurs, afin de maintenir le niveau constant dans les réacteurs 2 et 6, ceux-ci comportent également des conduites de "purge" 19 et 20 qui permettent d'éliminer les solutions aqueuses partiellement épuisées. Enfin, l'installation comprend une conduite 21 qui permet de relier les conduites 17 et 20 de façon à pouvoir renvoyer de façon continue ou discontinue la totalité ou une partie de l'effluent aqueux du réacteur 6 dans le réacteur 2.

L'installation peut être réalisée entièrement ou partiellement en tout matériau approprié, recouvert ou non de composés polymériques permettant d'améliorer la résistance des matériaux à certains des réactifs mis en oeuvre. Habituellement, la totalité de l'installation est réalisée en acier inoxydable.

Le chlorure de vinyle récupéré à la sortie du deuxième réacteur peut être utilisé dans toute fabrication et plus particulièrement pour la fabrication d'homopolymères à grande pureté.

Enfin, bien que l'invention concerne l'épuration de chlorure de vinyle en composés de type ester insaturé présentant une structure d'ester d'énol, il est évident qu'elle peut également conduire sans autre modification fondamentale à l'épuration d'autres halogénures d'alkyle du même type tels que le chlorure de vinylidène, fluorure de vinyle et fluorure de vinylidène ainsi qu'à l'épuration de ces composés en impuretés cétoniques ou aldéhydes d'une autre origine que l'ester d'énol.

L'invention est illustrée par les exemples suivants.

## Exemple 1

On opère dans une installation telle qu'illustrée à la figure 1 dans laquelle la colonne 2 et la colonne 6 ont un diamètre de 65 mm, une hauteur de 550 mm et un volume en liquide de 400 cm$^3$.

La colonne 2, maintenue par refroidissement à une température de 6°C, contient une solution de NaOH 1 molaire et la composition du pied de la colonne 6, maintenue à 25°C, est constituée d'une solution de NaBH$_4$ 1 molaire dans une solution aqueuse de NaOH 2,5 molaire.

On introduit dans cette installation, via la conduite 1 avec un débit de gaz d'environ 145 lN/h (i.e. litres normaux mesurés à 0°C et à pression atmosphérique/heure), du chlorure de vinyle récupéré d'une installation de copolymérisation avec de l'acétate de vinyle.

Cinq essais effectués avec des teneurs en acétate de vinyle comprises entre 360 (essai 3) et 47.000 ppm vol (essai 5) ont conduit aux résultats et observations récapitulés au Tableau 1.

Tableau 1

| | NUMERO DE L'ESSAI | | | | |
|---|---|---|---|---|---|
| | 1 | 2 | 3 | 4 | 5 |
| **Conditions des essais** | | | | | |
| -Réacteur alcalin : | | | | | |
| NaOH en mol/l | 1 | 1 | 1 | 1 | 1 |
| Volume en cm$^3$ | 400 | 400 | 400 | 400 | 400 |
| Température en °C | 6 | 6 | 6 | 6 | 6 |
| -Réacteur à : | | | | | |
| NaBH$_4$ en mol/l | 1 | 1 | 1 | 1 | 1 |
| NaOH en mol/l | 2,5 | 2,5 | 2,5 | 2,5 | 2,5 |
| Volume en cm$^3$ | 400 | 400 | 400 | 400 | 400 |
| Température en °C | 25 | 25 | 25 | 25 | 25 |
| -Débit de gaz traité : en 1N/h | 146,1 | 145,6 | 145,3 | 146,3 | 142,2 |
| -Durée de l'essai : | 1,5 | 1,75 | 2,3 | 1,3 | 0,83 |
| **Analyses par C.P.V.** * | | | | | |
| -Entrée du réacteur alcalin (colonne 2) : Acétate de vinyle en ppm vol | 1900 | 650 | 360 | 8.400 | 47.000 |
| -Entrée du réacteur à NaBH$_4$ (colonne 6) : Acétate de vinyle en ppm vol | < 1 | 3,1 | < 1 | < 1 | 10 |
| Acétaldéhyde en ppm vol | 220 | 140 | 46 | 231 | 1.700 |
| -Sortie du réacteur à NaBH$_4$ (colonne 6) : Acétate de vinyle en ppm vol | < 1 | < 1 | < 1 | < 1 | < 1 |
| Acétaldéhyde en ppm vol | < 1 | < 1 | < 1 | < 1 | < 1 |
| Ethanol en ppm vol | 37 | 36 | 53 | 22 | 229 |

5

| | NUMERO DE L'ESSAI | | | | |
|---|---|---|---|---|---|
| | 1 | 2 | 3 | 4 | 5 |
| **Résultats** | | | | | |
| Conversion de l'acétate de vinyle au réacteur alcalin en % | 100 | 99,5 | 100 | 100 | 99,98 |
| Conversion de l'acétate de vinyle au réacteur NaBH$_4$ en % | – | 100 | – | – | 100 |
| Acétaldéhyde sortie réacteur alcalin par rapport à l'acétate de vinyle mis en oeuvre en % mol. | 11,6 | 21,5 | 12,8 | 2,75 | 3,6 |
| Conversion de l'acétal-déhyde sortie réacteur à NaBH$_4$ en % mol. | 100 | 100 | 100 | 100 | 100 |

\* = Chromatographie en phase vapeur

On peut conclure au vu des résultats ci-dessus que le procédé de l'invention permet d'éliminer d'une façon quantitative l'acétate de vinyle présent dans le chlorure de vinyle et que celui-ci a dès lors une pureté permettant son recyclage à tout procédé y compris à l'homopolymérisation.

Exemple 2

On opère dans une installation et dans des conditions de température identiques à celles de l'exemple 1 mais avec une solution alcaline de NaOH 0,5 M dans la colonne 2 et une solution de NaBH$_4$ à 0,1 M dans une solution aqueuse de NaOH 2,5 M dans la colonne 6.

L'essai est réalisé avec des teneurs en acétate de vinyle de 23.000 ppm volume dans le chlorure de vinyle gazeux envoyé avec un débit gazeux d'environ 60 IN/h pour 400 cm$^3$ de pied liquide.

A l'entrée du réacteur à NaBH$_4$, le chlorure de vinyle contient 2318 ppm d'acétaldéhyde pour 18 ppm d'acétate de vinyle et, à la sortie de ce réacteur, on observe des teneurs inférieures à 1 ppm volume de ces deux impuretés avec une apparition de seulement 39 ppm d'éthanol.

Exemple 3

On opère dans des conditions identiques à celles de l'exemple 2 mais avec une solution alcaline de NaOH

6

0,5 M dans la colonne 2.

L'installation utilisée quant à elle est celle des exemples 1 et 2 modifiée de façon à permettre le recyclage d'une partie du liquide de la colonne 6 vers la colonne 2.

L'essai est réalisé avec un débit de recyclage d'une partie du liquide partiellement épuré de la colonne 6 vers la colonne 2 de 4 cm³/heure pendant 1 semaine via les conduites 20, 21 et 17 et les niveaux sont maintenus constants dans les réacteurs 2 et 6 par ajustement des débits d'alimentation en solutions fraîches.

On observe au cours de cet essai, outre les résultats déjà discutés à l'exemple 2, que l'hydrure recyclé à la colonne 2 a quasiment complètement disparu 5 minutes après le début de l'expérience et que tout au long de l'expérience, le milieu liquide de la colonne 2 reste clair et parfaitement liquide et qu'il n'y a pas formation de précipités dans cette colonne, ce qui n'est pas le cas des expériences des exemples 1 et 2 dans lesquelles on observe la formation de produits solides colorant le milieu réactionnel et formant des précipités.

## Revendications

1. Procédé d'épuration de chlorure de vinyle en composés de type ester insaturé présentant une structure d'ester d'énol, caractérisé en ce que le chlorure de vinyle impur subit un traitement comprenant une étape de lavage alcalin suivie ou concomitante à une étape de traitement avec un hydrure.

2. Procédé selon la revendication 1, caractérisé en ce que l'hydrure est un borohydrure.

3. Procédé selon les revendications 1 ou 2, caractérisé en ce que l'étape de lavage alcalin est réalisée avec une solution aqueuse d'une base dérivée de métaux alcalins ou alcalino-terreux.

4. Procédé selon les revendications 1 à 3, caractérisé en ce que l'étape de lavage alcalin est suivie d'une étape de traitement avec du borohydrure de sodium.

5. Procédé selon la revendication 4, caractérisé en ce qu'une partie ou la totalité de l'effluent aqueux de la deuxième étape est recyclée au réacteur de lavage alcalin.

6. Procédé selon l'une quelconque des revendications 1 à 5, caractérisé en ce que l'ester insaturé présent dans le chlorure de vinyle impur est l'acétate de vinyle.

## Patentansprüche

1. Verfahren zur Reinigung von Vinylchlorid von Verbindungen vom Typ ungesättiger Ester, die eine Enolester-Struktur aufweisen, dadurch gekennzeichnet, daß das unreine Vinylchlorid einer Behandlung unterzogen wird, die eine Stufe einer alkalischen Waschung gefolgt von oder gleichzeitig mit einer Stufe der Hydrid-Behandlung umfaßt.

2. Verfahren nach Anspruch 1, dadurch gekennzeichnet, daß das Hydrid ein Borhydrid ist.

3. Verfahren nach den Ansprüchen 1 oder 2, dadurch gekennzeichnet, daß die Stufe der alkalischen Waschung mit einer wässrigen Lösung einer Base, abgeleitet von Alkali- oder Erdalkalimetallen, durchgeführt wird.

4. Verfahren nach den Ansprüchen 1 bis 3, dadurch gekennzeichnet, daß die Stufe der alkalischen Waschung gefolgt wird von einer Stufe der Behandlung mit Natriumborhydrid.

5. Verfahren nach Anspruch 4, dadurch gekennzeichnet, daß ein Teil oder das gesamte wässrige Abwasser der zweiten Stufe zum Reaktor der alkalischen Waschung zurückgeführt wird.

6. Verfahren nach einem der Ansprüche 1 bis 5, dadurch gekennzeichnet, daß der in dem unreinen Vinylchlorid anwesende, ungesättigte Ester Vinylacetat ist.

**Claims**

1. Process for the purification of vinyl chloride from compounds of unsaturated ester type exhibiting an enol ester structure, characterised in that the impure vinyl chloride undergoes a treatment comprising an alkaline washing stage followed or accompanied by a stage of treatment with a hydride.

2. Process according to Claim 1, characterised in that the hydride is a borohydride.

3. Process according to Claims 1 or 2, characterised in that the alkaline washing stage is carried out with an aqueous solution of a base derived from alkali or alkaline-earth metals.

4. Process according to Claims 1 to 3, characterised in that the alkaline washing stage is followed by a stage of treatment with sodium borohydride.

5. Process according to Claim 4, characterised in that a part or all of the aqueous effluent from the second stage is recycled to the alkaline washing reactor.

6. Process according to any one of Claims 1 to 5, characterised in that the unsaturated ester present in the impure vinyl chloride is vinyl acetate.